(19) Europäisches Patentamt
European Patent Office
Office européen des brevets

(11) Veröffentlichungsnummer: **0 359 043**
**A1**

# (12) EUROPÄISCHE PATENTANMELDUNG

(21) Anmeldenummer: **89116070.7**

(51) Int. Cl.5: **C07C 59/215 , C07C 51/48 , C07C 69/716**

(22) Anmeldetag: **31.08.89**

(30) Priorität: **13.09.88 DE 3831071**

(43) Veröffentlichungstag der Anmeldung:
**21.03.90 Patentblatt 90/12**

(84) Benannte Vertragsstaaten:
**CH DE GB LI**

(71) Anmelder: **BASF Aktiengesellschaft**
**Carl-Bosch-Strasse 38**
**D-6700 Ludwigshafen(DE)**

(72) Erfinder: **Paust, Joachim, Dr.**
**Ringstrasse 3**
**D-6708 Neuhofen(DE)**
Erfinder: **von Deessen, Ulrich, Dr.**
**Im Lammsbauch 33**
**D-6720 Speyer(DE)**
Erfinder: **Ernst, Hansgeorg, Dr.**
**Bussardweg 62**
**D-6720 Speyer(DE)**
Erfinder: **Schaper, Michael, Dr.**
**Mundenheimer Strasse 160**
**D-6700 Ludwigshafen(DE)**

## (54) Verfahren zur Isolierung von 2-Keto-polyphydroxy-C6-carbonsäuren, insbesondere von 2-Keto-L-gulonsäure aus wässrigen Fermentationsausträgen.

(57) Verfahren zur Extraktion von 2-Keto-polyhydroxy-$C_6$-carbonsäuren, insbesondere von 2-Keto-L-gulonsäure, aus wäßrigen Fermentationsausträgen, das dadurch gekennzeichnet ist, daß man

a) einen von der Biomasse befreiten, die 2-Keto-polyhydroxy-$C_6$-carbonsäure als Calciumsalz enthaltenden Fermentationsaustrag unter einem $CO_2$-Druck von 10 bis 60 bar, in Gegenwart von 2 bis 6 Molequivalenten, bezogen auf die in dem Fermentationsaustrag enthaltene 2-Keto-polyhydroxy-$C_6$-carbonsäure, an einem langkettigen lipophilen Amin mit insgesamt 15 bis 40 C-Atomen und mit 1,5 bis 2,5 Gew.-Teilen, bezogen auf den Fermentationsaustrag, eines $C_4$- bis $C_8$-Alkanols bei Temperaturen zwischen 10 und 80 °C, vorzugsweise 55 bis 65 °C extrahiert,

b) das ausgefallene Calciumcarbonat entfernt und

c) das erhaltene 2-Keto-polyhydroxy-$C_6$-carbonsäure-Amine-Addukt in die freie Carbonsäure oder deren Derivat spaltet bzw. überführt. Als Amine werden vorzugsweise Trioctylamin oder Tridodecylamin verwendet, als Alkanole vorzugsweise Butanole.

EP 0 359 043 A1

# Verfahren zur Isolierung von 2-Keto-polyhydroxy-$C_6$-carbonsäuren, insbesondere von 2-Keto-L-gulonsäure aus wäßrigen Fermentationsausträgen

Die vorliegende Erfindung betrifft die Isolierung von auf fermentativem Wege hergestellten 2-Keto-polyhydroxy-$C_6$-carbonsäuren, insbesondere von so hergestellter 2-Keto-L-gulonsäure aus wäßrigen Fermentationsausträgen mittels Extraktion in Gegenwart von Aminen.

Stand der Technik:

2-Keto-L-gulonsäure ist ein zentrales Zwischenprodukt bei der Herstellung von Vitamin C. Vitamin C wurde bisher weltweit konventionell nach dem sogenannten Reichsteinverfahren hergestellt. Bei diesem Verfahren handelt es sich um einen vielstufigen Prozeß, der vor allem aufgrund der aufwendigen Schutzgruppenchemie nicht mehr zeitgemäß ist.

Aus diesem Grunde hat es nicht an Versuchen gefehlt, Verfahren zur Herstellung des begehrten Vitamin C auf biotechnologischer Basis zu entwickeln, die weniger Reaktionsschritte aufweisen und bei denen die Problematik geeigneter Schutzgruppen vermieden wird. Allen bisher bekannten wichtigen biotechnologischen Verfahren gemeinsam ist die Verwendung von D-Glucose als Ausgangsverbindung und die Bildung von 2-Keto-L-gluconsäure (2-KGS) als Vitamin-C-Vorstufe. Bekannte biotechnologische Wege:

a) Glucose → 2,5-Diketogluconsäure → 2-KGS → Vitamin C

b) Glucose → L-Sorbose → 2-KGS → Vitamin C.

Da die 2-KGS in beiden Fällen durch Fermentation erzeugt wird, findet man sie in wäßriger Lösung zusammen mit Biomasse vor. Für die Gewinnung reiner L-Ascorbinsäure (Vitamin C) ist aber eine Isolierung der 2-KGS bzw. eine Isolierung von deren Derivaten aus den Fermentationsausträgen unumgänglich.

Bei den meisten bisher bekannten fermentativen Verfahren wird der für die Fermentation geeignete pH-Wert durch Zufügen eines Calciumsalzes eingestellt, wodurch 2-KGS in Form ihres Calciumsalzes in der wäßrigen Fermentationsbrühe anfällt.

Nach Shionogi (vgl. JA-OS 52 066684) wird aus dieser Lösung das Calcium durch Zugabe von Natriumcarbonat als Calciumcarbonat gefällt und abfiltriert, das Filtrat eingedampft und durch Zusatz von Methanol 2-KGS als Natriumsalz ausgefällt. Die Ausbeute beträgt hierbei ca. 87 %. Durch Behandeln des Natriumsalzes mit HCl in einem Lösungsmittel werden nacheinander NaCl und Vitamin C erhalten (vgl. EP-A 91 134). Die Umlagerung verläuft mit 72 % Ausbeute.

Gemäß Verfahren von Takeda (vgl. JA-OS 75 022 113) erfolgt die Isolierung von 2-KGS durch Ionenaustausch. Nach Veresterung der Säure wird mit Natriummethylat zum Natriumascorbat in 86%iger Ausbeute umgelagert.

Nachteilig bei allen diesen Verfahren ist, daß große Fermentationsvolumina bewegt werden müssen und daß Natriumketogulonat mit HCl zur Ascorbinsäure sauer umgelagert wird, wobei eine große Menge Salz als unerwünschtes Nebenprodukt entsteht. Entsprechend wird bei der alkalischen Umlagerung des Esters Natriumascorbat erzeugt dessen Überführung in L-Ascorbinsäure ebenfalls die Bildung großer Mengen Salz mit sich bringt, was aus umwelttechnischen und Kostengründen unerwünscht ist.

Es war daher die Aufgabe der Erfindung, ein möglichst einfaches Verfahren zur Isolierung von 2-KGS aus wäßrigen Fermentationsausträgen zu entwickeln, bei dem die Nachteile des Standes der Technik nicht auftreten, bei dem insbesondere die Bildung großer Mengen an Salzen entfällt.

Es wurde nun überraschenderweise gefunden, daß 2-KGS in Gegenwart von langkettigen lipophilen Aminen mit 15 bis 40 C-Atomen in einfacher Weise durch Extraktion mit höheren Alkanolen aus der Fermentationsbrühe abgetrennt werden kann, wenn man aus dem von Biomasse befreiten Fermentationsaustrag unter den Extraktionsbedingungen durch Einleiten von $CO_2$ unter Druck in situ die enthaltenen Calciumionen abgetrennt, d.h. also unter $CO_2$-Druck arbeitet. Aus dem gebildeten 2-KGS-Amin-Addukt können dann auf einfache Weise 2-KGS, 2-KGS-Salz oder 2-KGS-Ester gewonnen werden.

Aus US 4,444,881 ist zwar schon die Extraktion von Carbonsäuren aus deren wäßrigen Lösungen der entsprechenden Calciumsalze mit Amincarbonaten bekannt, jedoch handelt es sich hierbei

1) um einfache Säuren, wie Propionsäure, Buttersäure, Milchsäure und Citronensäure und

2) werden als geeignete Amine, insbesondere tertiäre Amine, wie Tributylamin und Dicyclohexylmethylamin, genannt. Die langkettigen Amine, die für die Extraktion von 2-Ketogulonsäure besonders gut geeignet sind, wie Trioctylamin und Tridodecylamin werden ausgeschlossen. Bevorzugte Extraktionsmittel dieses Verfahrens sind chlorierte Kohlenwasserstoffe, wie Chloroform.

Die Anwendung der bekannten Extraktionsbedingungen auf die Gewinnung von 2-KGS aus Fermentationsausträgen lieferte keine oder nur sehr schlechte, wirtschaftlich unakzeptable Ausbeuten an 2-KGS.

Dies dürfte einerseits in dem üblicherweise zu starker Emulsionsbildung führenden stark polaren Charakter von 2-KGS

COOH / =O / HO— / —OH / HO— / OH    I

OH / HO / O / COOH / OH / OH    II

begründet sein (vgl. Polyhydroxyketocarbonsäure I), andererseits dadurch, daß 2-KGS aufgrund der Oxo-Cyclo-Tautomerie (vgl. II mit zuckerähnlicher Struktur und zuckerähnlichem Verhalten) zahlreiche Nebenreaktionen eingehen kann.

Gegenstand der Erfindung ist ein Verfahren zur Extraktion von 2-Keto-polyhydroxy-$C_6$-carbonsäuren, insbesondere von 2-KGS, aus wäßrigen Fermentationsausträgen, das dadurch gekennzeichnet ist, daß man

a) einen von Biomasse befreiten, die 2-Keto-polyhydroxy-$C_6$-carbonsäure als Calciumsalz enthaltenden Fermentationsaustrag unter einem $CO_2$-Druck von 10 bis 60 bar in Gegenwart von 2 bis 6, vorzugsweise 3,5 bis 5 Molequivalenten, bezogen auf die in dem Fermentationsaustrag enthaltene 2-Keto-polyhydroxy-$C_6$-carbonsäure, an einem Amin mit insgesamt 15 bis 40 C-Atomen mit 1,5 bis 2,5, vorzugsweise 1,6 bis 2 Gew.-Teilen, bezogen auf das Amin eines $C_4$-bis $C_8$-Alkanols bei Temperaturen zwischen 20 und 80 °C, vorzugsweise 55 bis 65 °C extrahiert,

b) das ausgefallene Calciumcarbonat abtrennt und

c) das erhaltene 2-Keto-polyhydroxy-$C_6$-carbonsäure-Addukt in die freie Carbonsäure oder deren Derivat spaltet bzw. überführt.

Als 2-Keto-polyhydroxy-$C_6$-carbonsäure, die erfindungsgemäß aus sie enthaltenden Fermentationsausträgen isoliert werden können, kommen insbesondere 2-Keto-L-gulonsäure, 2-Keto-D-gluconsäure und 2-Keto-galaktonsäure in Betracht. Von besonderer Bedeutung ist das Verfahren für die Isolierung der für die Herstellung von Vitamin C dringend benötigten 2-Keto-L-gulonsäure. Die Ketocarbonsäuren fallen bei ihrer fermentativen Herstellung im allgemeinen in Fermentationsbrühen an, die zwischen 1 und 20 Gew.-%. vorzugsweise etwa 7 - 12 Gew.-% der Carbonsäure enthalten. Bei der erfindungsgemäßen Extraktion unter $CO_2$-Druck kann man wesentlich konzentriertere Lösungen einsetzen. Sehr vorteilhaft arbeitet man mit Lösungen, die etwa 30 Gew.-% an 2-KGS enthalten. Verdünntere Fermentationsausträge können dementsprechend eingeengt werden.

Vor dem Extraktionsprozeß wird aus diesen Fermentationsbrühen die Biomasse in an sich bekannter Weise durch Abfiltrieren oder Zentrifugieren abgetrennt.

Zur Extraktion werden dem Fermentationsaustrag 2 bis 6, vorzugsweise etwa 3,5 bis 5 Molequivalente, bezogen auf die in dem Fermentationsaustrag enthaltene 2-Keto-polyhydroxy-$C_6$-carbonsäure, des Amins zugefügt. Als Amine kommen langkettige, lipophile Amine mit aliphatischen, cycloaliphatischen und aromatischen Resten mit insgesamt 15 bis 40 C-Atomen in Betracht. Genannt seien insbesondere Trioctylamin, Trilaurylamin, Tri-tridecylamin, Tricaprylamin, käufliche Gemische aus Trilauryl- und Dilaurylamin (Tridodecylamin) und Ditridecylamin.

Mit besonderem Vorteil arbeitet man mit handelsüblichem Trioctylamin, Tridodecylamin oder Ditridecylamin.

Als eigentliche Extraktionsmittel dienen $C_4$- bis $C_8$-Alkanole, wie n-Butanol, iso-Butanol, Pentanole, Hexanole, Heptanole und Octanole, insbesondere Methylisobutylcarbinol, n-Butanol und iso-Butanol. Die Alkanole verwendet man in Mengen von 1,5 bis 2,5, vorzugsweise 1,6 bis 2 Gew.-Teilen, bezogen auf das lipophile Amin. Die verwendete Gesamtmenge an langkettigem Amin und Alkanol richtet sich im allgemeinen nach der Zusammensetzung des Fermentationsaustrages. Durch einfache Vorversuche kann für jeden Fall festgestellt werden, bei welcher Menge eine gute Trennung erzielt werden kann und keine Emulsionsbildung auftritt. Größenordnungsmäßig arbeitet man mit einem Verhältnis von Fermenteraustrag zu Extraktionsgemisch von etwa 0,15/1 bis 0,2/1, vorzugsweise 0,16 bis 0,18/1.

Bei dem erfindungsgemäßen Verfahren fällt Calciumcarbonat aus dem Reaktionsgemisch aus, das nach Abfiltrieren wieder in die Fermentationsstufe zur pH-Wert-Kontrolle zurückgeführt werden kann.

Hierdurch wird der bei einer gesonderten Abtrennung von Calcium auftretende Zwangsanfall von Calciumsulfat vermieden.

Zur Durchführung des Verfahrens wird die Calciumpolyhydroxy-$C_6$-carbonsäure, insbesondere Calcium-

Ketogulonat enthaltende Lösung, bzw. der von Biomasse befreite und ggf. aufkonzentrierte Fermentationsaustrag mit dem Gemisch aus dem langkettigen Amin und dem Alkanol versetzt, im Autoklaven mit 10 bis 60 bar $CO_2$ begast und bei einer Temperatur von 20 bis 80 °C, vorzugsweise 55 bis 65 °C über einen Zeitraum von 5 bis 60 Stunden (h), vorzugsweise etwa 10 bis 15 h, gerührt.

Die Extraktion kann im Chargen-(batch)-Betrieb, aber mit Vorteil auch kontinuierlich (z.B. in einem Mixer-Settler) durchgeführt werden.

Nach dem Abtrennen des ausgefallenen Calciumcarbonats und der Phasentrennung wird aus der das Wertprodukt als Amin-Addukt enthaltenden alkoholischen Phase unter vermindertem Druck der Alkohol destillativ entfernt. Bei Verwendung von Butanolen wird das 2-KGS-Amin-Addukt beispielsweise aus der Oberphase durch Abdestillieren des Alkohols bei etwa 10 bis 100 mbar, vorzugsweise 15 bis 25 mbar, mit Hilfe eines Rotationsverdampfers bei 20 bis 50 °C, vorzugsweise etwa 30 °C isoliert. Der abdestillierte Alkohol kann erneut zur Extraktion verwendet werden. Die Unterphase kann einer weiteren Extraktion zugeführt werden.

Das bei der beschriebenen erfindungsgemäßen Extraktion anfallende 2-Keto-polyhydroxy-$C_6$-Carbonsäure-Amin-Addukt kann auf verschiedene Weise weiterverarbeitet werden. Der Einfachheit halber beschreiben wir im einzelnen die Weiterverarbeitung der besonders wichtigen 2-KGS.

1. Zur Gewinnung von freier 2-Keto-L-gulonsäure kann man das 2-KGS-Amin-Addukt mit etwa 3 bis 8 Gew.-Teilen Test-benzin (Kp 155 bis 185 °C), bezogen auf das 2-KGS-Amin-Addukt, versehen und bei Temperaturen von 60 bis 120 °C, vorzugsweise etwa 90 °C, im Reaktionsgefäß für 10 bis 60 Minuten (min.), vorzugsweise etwa 30 min. rühren. Die ausfallenden 2-KGS-Kristalle werden heiß abgesaugt. Man erzielt hierbei Ausbeuten von etwa 94 % Das Amin kann in den Prozeß zurückgeführt werden.
Das 2-KGS-Amin-Addukt kann aber auch mit Wasser bei Raumtemperatur (RT) in die freie 2-KGS und Amin gespalten werden. Hierzu wird das erhaltene 2-KGS-Amin-Addukt durch Behandeln mit 2 bis 20, vorzugsweise etwa 10 Gew.-Teilen Wasser, bezogen auf das Addukt, bei Temperaturen von 5 bis 50 °C, vorzugsweise etwa 20 °C in die freie 2-KGS und das eingesetzte Amin gespalten, wozu im allgemeinen Reaktionszeiten von etwa 10 bis 60 Minuten notwendig sind.

2. Zur Gewinnung von Alkalisalzen der 2-KGS wird das 2-KGS-Amin-Addukt im allgemeinen mit der 2- bis 5-fachen, vorzugsweise etwa 3fachen Gewichtsmenge an Wasser versetzt und das erhaltene Gemisch anschließend mit einer Alkalihydroxidlösung titriert bis ein pH-Wert zwischen 9 und 12, vorzugsweise etwa 10 bis 11 erreicht ist. Das Amin kann aus dem erhaltenen Gemisch beispielsweise mit Hexan extrahiert und nach destillativem Entfernen des Hexans erneut verwendet werden. Aus der wäßrigen Phase kann das Alkalisalz der 2-KGS durch Eindampfen in etwa 97%iger Ausbeute erhalten werden.

3. Zur Gewinnung von 2-KGS-Estern wird das 2-KGS-Amin-Addukt mit der 1,5-bis 4-fachen Gewichtsmenge einer etwa 2,5 N Lösung HCl in einem $C_1$- bis $C_5$-Alkanol, vorzugsweise in Methanol und einem inerten Lösungsmittel, wie Methylenchlorid versetzt und mehrere Stunden (h), vorzugsweise etwa 12 h bei RT gerührt. Hierbei kristallisiert der sich bildende Ester aus dem Reaktionsgemisch aus und kann durch Filtration abgetrennt werden. Die Ausbeute an 2-KGS-methylester beträgt bei diesem Verfahren etwa 95,9 % der Theorie.

Mit Hilfe des erfindungsgemäßen Verfahrens können 2-Keto-polyhydroxy-$C_6$-carbonsäuren, insbesondere die für die Herstellung von Vitamin C sehr begehrte 2-Keto-L-gulonsäure, auf relativ einfache und wirtschaftlich vorteilhafte Weise aus wäßrigen Fermentationsausträgen in reiner Form oder in Form von ihren Salzen oder Estern isoliert werden. Die so erhaltene 2-KGS bzw. deren Derivate weisen eine hohe Reinheit auf und können auf bekannte Weise in Vitamin C überführt werden.

Die folgenden Beispiele sollen das erfindungsgemäße Verfahren erläutern. Die Versuche sind noch nicht optimiert.

Beispiel 1

Zu 15 ml einer wäßrigen Lösung enthaltend 3,8g 2-KGS (gemäß HPLC) und 0,37 g Calciumionen (Titration mi Titriplex) wurde ein Gemisch aus 60 g Isobutanol und 36 g (100 mMol, entsprechend 5,2 Molequivalenten) Trioctylamin gegeben, die Mischung in einen Autoklaven gefüllt, 60 bar $CO_2$ aufgepreßt und das Reaktionsgemisch 12 h bei 60 °C gerührt.

Anschließend wurde das erhaltene 3-Phasengemisch aus dem Autoklaven entnommen, das ausgefallene $CaCO_3$ abgesaugt und getrocknet (0,24 g). Die Oberphase wurde nach Einengen am Rotationsverdampfer und Aufnehmen in einem Gemisch aus 100 ml Hexan und 50 ml Wasser mit 0,1 nNaOH gegen Phenolphthalein titriert.

Beispiel 2

In einen Autoklaven wurde zu 15 ml einer wäßrigen Lösung enthaltend 3,9g 2-KGS (gemäß HPLC) und 0,37 g Calciumionen (nach Titration mit Titriplex) ein Gemisch aus 61 g iso-Butanol und 24 g (69 mMol, entsprechend 3,5 Molequivalenten) Trioctylamin gegeben und das Reaktionsgemisch 60 h bei 60 °C und 60 bar $CO_2$-Druck gerührt. Aufarbeitung analog Beispiel 1 einer Extrakti onsausbeute von 37,9 % Die wäßrige Unterphase enthielt noch 1,6g 2-KGS.

Beispiel 3

In einem Autoklaven wurde zu 15 ml einer wäßrigen Lösung enthaltend 3,9g 2-KGS und 0,37 g Calciumionen eine Mischung aus 61 g n-Butanol und 36 g (100 mMol, entsprechend 5,2 Molequivalenten) an Trioctylamin gegeben und das Reaktionsgemisch 12 h bei 60 °C und 60 bar $CO_2$-Druck gerührt. Aufarbeitung analog Beispiel 1 ergab 0,35 g $CaCO_3$, 1,44 g 2-KGS, entsprechend einer Extrakti onsausbeute von 36,9 %. Die Unterphase enthielt noch 2,24 g 2-KGS.

**Ansprüche**

1. Verfahren zur Extraktion von 2-Keto-polyhydroxy-$C_6$-carbonsäuren aus wäßrigen Fermentationsausträgen, dadurch gekennzeichnet, daß man

a) einen von der Biomasse befreiten, die 2-Keto-polyhydroxy-$C_6$-carbonsäure als Calciumsalz enthaltenden Fermentationsaustrag unter einem $CO_2$-Druck von 10 bis 60 bar, in Gegenwart von 2 bis 6 Molequivalenten, bezogen auf die in dem Fermentationsaustrag enthaltene 2-Keto-polyhydroxy-$C_6$-carbonsäure, an einem Amin mit insgesamt 15 bis 40 C-Atomen mit 1,5 bis 2,5 Gew.-Teilen, bezogen auf das Amin eines $C_4$- bis $C_8$-Alkanols bei Temperaturen zwischen 20 und 80 °C extrahiert,

b) das ausgefallene Calciumcarbonat abtrennt und

c) das erhaltene 2-Keto-polyhydroxy-$C_6$-carbonsäure-Amin-Addukt in die freie Carbonsäure oder deren Derivat spaltet bzw. überführt.

2. Verfahren gemäß Anspruch 1, dadurch gekennzeichnet, daß man einen 2-Keto-L-gulonsäure enthaltenden Fermentationsaustrag einsetzt.

3. Verfahren gemäß Anspruch 2, dadurch gekennzeichnet, daß man den Fermentationsaustrag in Gegenwart von 3,5 bis 5 Molequivalenten, bezogen auf die in dem Fermentationsaustrag enthaltene 2-Keto-L-gulonsäure, an einem langkettigen Amin extrahiert.

4. Verfahren gemäß Anspruch 2, dadurch gekennzeichnet, daß man den Fermentationsaustrag mit etwa 1,6 bis 2 Gew.-Teilen eines $C_4$- bis $C_8$-Alkanols, bezogen auf das Amin extrahiert.

5. Verfahren gemäß Anspruch 1, dadurch gekennzeichnet, daß man als Amin Trioctylamin, Tridodecylamin oder Ditridecylamin verwendet.

6. Verfahren gemäß Anspruch 1, dadurch gekennzeichnet, daß man als $C_4$- bis $C_8$-Alkanol ein Butanol verwendet.

7. Verfahren gemäß Anspruch 2, dadurch gekennzeichnet, daß man das erhaltene 2-Keto-L-gulonsäure-Amin-Addukt durch Erhitzen auf 60 bis 120 °C in freie 2-Keto-L-gulonsäure und das eingesetzte Amin zurückspaltet.

8. Verfahren gemäß Anspruch 2, dadurch gekennzeichnet, daß man das erhaltene 2-Keto-L-gulonsäure-Amin-Addukt durch Behandeln mit 2 bis 20 Gew.-Teilen Wasser, bezogen auf das Addukt, bei Temperaturen von 5 bis 50 °C, in die freie 2-Keto-L-gulonsäure und das eingesetzte Amin spaltet.

9. Verfahren gemäß Anspruch 2, dadurch gekennzeichnet, daß man das erhaltene 2-Keto-L-gulonsäure-Amin-Addukt mit einem Alkali- oder Erdalkalihydroxid in das entsprechende Salz der 2-Keto-L-gulonsäure überführt.

10. Verfahren gemäß Anspruch 2, dadurch gekennzeichnet, daß man das erhaltene 2-Keto-L-gulonsäure-Amin-Addukt mit einem Chlorwasserstoffgas enthaltendem $C_1$- bis $C_5$-Alkanol, ggf. in einem inerten Lösungsmittel, in den entsprechenden Alkanolester der 2-Keto-L-gulonsäure überführt.

Europäisches Patentamt

# EUROPÄISCHER RECHERCHENBERICHT

Nummer der Anmeldung

EP 89116070.7

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | CH - A - 336 367 (PFIZER) * Gesamt * | 1,2 | C 07 C 59/215<br>C 07 C 51/48<br>C 07 C 69/716 |
| D,A | US - A - 4 444 881 (B. URBAS) * Gesamt * | 1,5,7,9 | |
| | | | RECHERCHIERTE SACHGEBIETE (Int. Cl.4) |
| | | | C 07 C 51/00<br>C 07 C 59/00<br>C 07 C 69/00 |

Der vorliegende Recherchenbericht wurde für alle Patentansprüche erstellt.

| Recherchenort | Abschlußdatum der Recherche | Prüfer |
|---|---|---|
| WIEN | 23-11-1989 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82